# EUROPEAN PATENT SPECIFICATION

(11) **EP 1 847 595 B1**
(45) Date of publication and mention of the grant of the patent: **06.01.2010**
(21) Application number: 07251672.7
(22) Date of filing: 20.04.2007
(51) Int. Cl.: G01N 33/68

(54) **Methods of assessing embryo viability**
Verfahren zur Beurteilung der Lebensfähigkeit von Embryos
Procédé pour évaluer la viabilité d'un embryon

(30) Priority: 20.04.2006 AU 2006902064
(43) Date of publication of application: 24.10.2007
(73) Proprietor: Northern Sydney and Central Coast Area Health Service, Gosford NSW 2250 (AU)
(72) Inventor: O'Neill, Christopher, Greenwich, New South Wales 2065 (AU)
(74) Representative: Thornton, Neil

(56) References cited:
- WO-A-01/23893
- WO-A-01/53518
- KATZ-JAFFE ET AL: "Analysis of protein expression (secretome) by human and mouse preimplantation embryos" FERTILITY AND STERILITY, ELSEVIER SCIENCE INC, NEW YORK, NY, US, vol. 86, no. 3, September 2006 (2006-09), pages 678-685, XP005633568 ISSN: 0015-0282
- ROUDEBUSH W E ET AL: "Embryonic platelet-activating factor: an indicator of embryo viability." HUMAN REPRODUCTION (OXFORD, ENGLAND) MAY 2002, vol. 17, no. 5, May 2002 (2002-05), pages 1306-1310, XP002444999 ISSN: 0268-1161

## Description

### Technical Field

The present invention relates generally to methods of assessing embryo viability, in particular assessing the viability of embryos produced by assisted reproductive technologies such as in vitro fertilization.

### Background of the Invention

Failure of survival of the embryo over the first weeks of its existence is considered to be a major cause of sub-fertility and infertility in mammals. This is particularly exemplified by embryos produced by assisted reproductive technologies (ART), including *in vitro* fertilization (IVF) and all related techniques. Not only is ART commonplace in human medicine, largely as a treatment for infertility, but it is also widely employed in agriculture to increase the rate of genetic gain in animals and to enhance fertility in high value animals such as beef cattle and horses. Embryos produced by ART are also the only source of human embryos used in the production of embryonic stem cell lines.

It has long been known that ART results in a characteristic phenotype of slow embryo development in vitro resulting in embryos with fewer cells and more cells undergoing death by apoptosis (see O'Neill, 1998). Upon transfer of cultured embryos there is a characteristic reduction in the rate of formation of normal fetuses (Bowman and McLaren, 1970). This phenotype is particularly severe in human embryos produced by ART. For the decade 1993-2002, human ART in Australia resulted in 9.1 live births per 100 embryos transferred (Bryant *et al.,* 2004). This inefficiency of ART makes the technology expensive, causing it to be prohibitive for many otherwise suitable agricultural purposes and a significant financial burden for human medicine. Furthermore, the low rates of embryo viability encourage the transfer of multiple embryos, creating a significant risk of multiple birth with consequent poor obstetric outcomes. It is generally recognized that these poor outcomes are a consequence of the adverse effects of embryo culture on the early embryo.

The ability to screen an embryo's developmental potential and to detect those embryos with the greatest viability after embryo culture would alleviate many of these problems associated with ART. Not only would this greatly contribute to improving the success, efficiency and utility of ART in medicine and agriculture, but may also lead to new therapeutic modalities.

Studies measuring aspects of embryo metabolism have been found to show a correlation with embryo viability, but have not found general use due to complexity and cost of assays and their generally low predictive power. A retrospective clinical study of a novel non-invasive method of embryo selection based on the depletion/appearance of amino acids in the culture medium showed that the turnover of Asn, Gly and Leu, was significantly correlated with a clinical pregnancy and live birth. These correlations were independent of known predictors, such as female age, basal levels of FSH, embryo cell number and embryo morphological grade (Brison *et al.,* 2004). In another study (Turner *et al.,* 1994), pyruvate uptake by embryos was related to their capacity for implantation. An association was also found between embryo morphology and pyruvate consumption. Morphologically good embryos were more likely to implant if they demonstrated an intermediate pyruvate uptake. No relationship was found between the type of infertility and pyruvate consumption of individual embryos. It was suggested that the ability of an embryo to implant is multifactorial and that both morphology and pyruvate uptake may be factors. The technical complexity of these assays and their relatively poor predictive power has meant that little progress has been made with their use as a routine assay of embryo viability. Further, whilst a positive association between the release of the autocrine embryotrophin platelet activating factor (Paf) and the developmental potential of an embryo has been found, the utility of the Paf assay has been constrained by its difficulty and reliability of extraction from embryo culture media. This makes its quantification and assay difficult, unreliable and time-consuming (O'Neill, 2005).

The preimplantation embryo produces survival factors that act in autocrine loops to drive the proliferation and survival of the early embryo. ART induces a number a stresses which activate stress pathways within the early embryo. This in turn leads to the production of a number of factors important in the survival of the embryo. For example, the important stress sensor, p53 is normally maintained at very low levels in the pre-implantation embryo. However it has been demonstrated that p53 expression is upregulated in embryos produced by ART and that p53 is a significant factor in the high rate of embryopathy following ART (WO 2005/019440).

WO 01/23893 describes a method for selecting an embryo for in vitro fertilization which includes detecting soluble human leukocyte antigen G (HLA-G) secreted by an embryo into an incubation medium.

Roudebush et al. (Human Reproduction Vol. 17, No. 5, pp. 1306-1310, 2002) describes a correlation between platelet-activating factor (PAF) levels in human embryo conditioned culture media and pregnancy outcome.

WO 01/53518 describes a method of assessing the viability of a cell comprising incubating the cell in a culture medium including a plurality of amino acids and determining the change in concentration in the medium of at least one amino acid.

WO 2005/001127 describes a method for the determination of the risk of a subject having or developing cancer or other condition associated with the expression of a particular polymorphism of the p53 allele, of its homolog or ortholog.

The present invention is predicated on the finding that several protein markers may released by a cultured embryo into its culture media and that the release of these factors is correlated with the viability of the embryo.

### Summary

Accordingly, in a first aspect there is provided a method for determining the viability of an embryo or an embryo precursor, the method comprising:
(a) culturing an embryo or an embryo precursor *in vitro* in culture medium;
(b) measuring the level of a marker in the culture medium; and
(c) comparing the measured level of the marker in the culture medium to at least one level of the marker which is indicative of the viability of the embryo and thereby determining the embryo viability.

In another aspect, there is provided a method of ranking an embryo or embryo precursor on the basis of its viability, the method comprising:
(a) culturing individually a plurality of embryos or embryo precursors *in vitro* in culture medium;
(b) measuring the level of a marker in the culture medium of each embryo or embryo precursor;
(c) comparing the measured level of the marker in the culture medium of each embryo to at least one level of the marker which is indicative of the viability of the embryo.

In one embodiment, the method comprises the step of identifying the embryo with the greatest viability.

In these aspects, the marker is p53. Use of Caᵥ₁.₂ or catalase as marker is also described.

In some embodiments of these aspects the embryo precursor is a fertilized egg, a zygote or another pre-embryonic entity. The embryo or embryo precursor may be produced by assisted reproductive technology, such as *in vitro* fertilization.

In some embodiments of these aspects the step (c) of comparing the measured level of the marker in the culture medium to at least one level of the marker which is indicative of the viability of the embryo comprises comparing the level of the marker in culture medium with the level of the marker from a control sample. The control sample may be a positive control comprising pooled culture media from embryos having high viability, or a negative control comprising pooled culture media from embryos having low viability.

In some embodiments of these aspects the step (c) of comparing the measured level of the marker in the culture medium to at least one level of the marker which is indicative of the viability of the embryo comprises comparing the level of the marker in the culture medium to the level of the marker in two or more control samples.

In one embodiment, the embryo is a human embryo.

Use of a kit for determining the viability of an embryo or embryo precursor is described, the kit comprising at least one agent for measuring the level of a marker in culture media. The marker may be p53, in which case the at least one agent may comprise an antibody to p53.

Also provided is a method of screening a candidate agent for the ability to modulate the viability of an embryo or embryo precursor, the method comprising contacting a first non-human embryo or non-human embryo precursor with the candidate agent, culturing the first embryo or embryo precursor in vitro in culture medium, culturing a second non-human embryo or non-human embryo precursor which has not been exposed to the candidate agent in vitro in culture medium, measuring the level of a marker which is indicative of the viability of the embryo in the culture medium of the first and the second embryo or embryo precursor, comparing the measured level of the marker in the culture medium of the first and the second embryo or embryo precursor and thereby determining whether the candidate agent modulates embryo viability.

In another aspect there is provided a method of screening a candidate agent for the ability to modulate the viability of an embryo or embryo precursor, the method comprising contacting a non-human embryo or non-human embryo precursor with the candidate agent, culturing the embryo or embryo precursor in vitro in culture medium, measuring the level of a marker which is indicative of the viability of the embryo in the culture medium, comparing the measured level of the marker in the culture medium with at least one level which is indicative of the viability of the embryo and thereby determining whether the candidate agent modulates embryo viability.

The marker in any of the above aspects is p53.

In certain embodiments of these screening methods, the candidate agent is a culture medium.

In some embodiments of these screening methods, the modulation of embryo viability is an increase in embryo viability.

In any of the aspects described above, the embryo may be a non-human embryo, such as an embryo of a non-human primate, equine, bovine, ovine, caprine, leporine, avian, feline, canine or a murine species.

### Definitions

In the context of this specification, the term "comprising" means "including principally, but not necessarily solely". Furthermore, variations of the word "comprising", such as "comprise" and "comprises", have correspondingly varied meanings.

As used herein the term "marker" refers to one or more cellular constituents or products produced by the developing embryo and released into the medium in which the embryo is being maintained or cultured. In the context of the present specification a marker is a polypeptide or oligopeptide or larger fragment thereof. As used herein the term "fragment" refers to polypeptide sequence comprising a constituent sequence of a full-length protein defined as a marker above. In terms of the polypeptide, the fragment possesses qualitative biological activity in common with the full length protein. A biologically active fragment of a marker protein may typically possess at least about 50% of the activity of the corresponding full length protein, more typically at least about 60% of such activity, more typically at least about 70% of such activity, more typically at least about 80% of such activity, more typically at least about 90% of such activity and more typically at least about 95% of such activity. As used herein the term "polypeptide" means a polymer which comprises as series of amino acids linked together by peptide bonds. A polypeptide as used herein may consist solely of amino acids, or may be a glycoprotein or a lipoprotein. It will be understood that in the context of this specification that reference to the detection or measuring of the level of a marker is not intended to exclude the detection or measuring of the levels of two or more markers.

As used herein the term "embryo or embryo precursor" refers to a zygote or post-zygotic derivatives of a fertilized egg. In a human, the embryonic stage is considered to begin approximately 2 weeks post fertilization. The term "embryo precursor" therefore refers to any entity in the pre-embryonic stage following fertilization of the egg. The term thus includes a fertilized egg and a zygote.

The term "expression" as used herein refers interchangeably to expression of a gene or gene product, including the encoded protein and to portions thereof. Expression of a gene product may be determined, for example, by Western blot or immunoassay using an antibody or antibodies that bind with the polypeptide. Accordingly, in the context of the present invention, expression may refer to the expression of a protein marker or a polypeptide fragment thereof.

In the context of this specification, the term "viability of an embryo" and "embryo viability" mean the likelihood of survival of an embryo. For the purposes of the present invention, embryo viability may be reflected in a number of indicators. For example increased embryo viability may result in any one or more of increased embryo implantation rates following *in vitro* fertilization, decreased pre- and post-implantation embryo lethality, increased clinical pregnancy rates or increased birth rates. An increase in the proportion of viable embryos may be exhibited in any one or more of an increase in the proportion of embryos developing into morphologically normal blastocysts, an increase in the number of cells per blastocyst and a decrease in the number of cells in the embryo undergoing apoptosis.

The term "detecting" a marker as used herein means the identification of the presence or absence of a marker. "Measuring" the level of a marker involves the quantification of amounts the marker. The quantification may involve assigning a numerical value of concentration, amount, or it may involve comparing the level of the marker with the level of a marker from a comparator sample, such as a control sample or the level provided by a standard curve. Accordingly "measuring" the level of a marker in an embryo culture medium may involve comparing the level of the marker in the medium to the level present in a pooled medium from embryos of known viability. Pooled embryos of known high viability may be readily generated by obtaining, for example, blastocysts collected fresh from the reproductive tract. Pooled embryos of low viability may be readily generated by, for example producing blastocysts via IVF techniques described herein.

### Brief Description of the Figures

The present invention will now be described by way of example with reference to the accompanying figures.

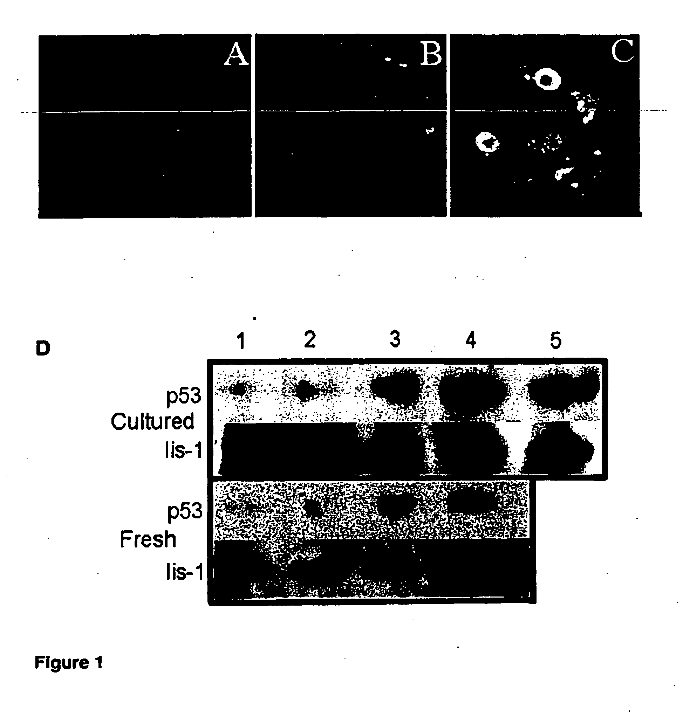

**Figure 1****. A to C** provides photomicrographs illustrating the immunolocalization of p53 in mouse blastocysts for embryos collected fresh from the reproductive tract (A), those fertilized in the reproductive tract but then cultured *in vitro* (B) and those produced by *in vitro* fertilization and then cultured *in vitro* (C). Embryos were cultured individually in 10 µL of media. Images were single confocal sections through each image. Staining, imaging, laser settings and image capture was performed under identical conditions for all embryos. **Figure 1** **D** provides a photograph of a western blot which illustrates an analysis of p53 expression by embryos. The figure illustrates the expression of p53 within preimplantation stage embryos (cultured C57BL6 embryos collected at the zygote stage and cultured for 24 h (1), 48 h (2), 72 h (3), 96 h (4)). Lis -1 is a constitutively expressed polypetide which was used as a loading control. Alternatively, embryos were collected fresh from the reproductive tract at the (1) zygote, (2) 2-cell, (3) morula, and (4) blastocyst stages, and their p53 expression assessed. A positive control for the expression of p53 was the analysis of - 1000 T47D breast cancer cells (5). Lis-1 is a constitutively expressed protein in the early embryo

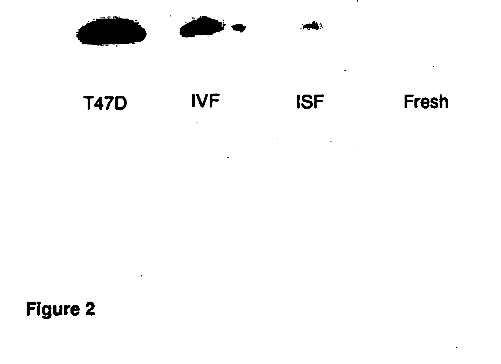

**Figure 2** provides a photograph of a western blot analysis of p53 within culture media resulting from culture of blastocysts produced by IVF, or embryos fertilized *in situ* and then cultured to blastocyst stage (ISF) or blastocysts collected fresh from reproductive tract and then placed in media for 6 h (Fresh). Media conditioned by 3,000 T47D cancer cells were used as a control.

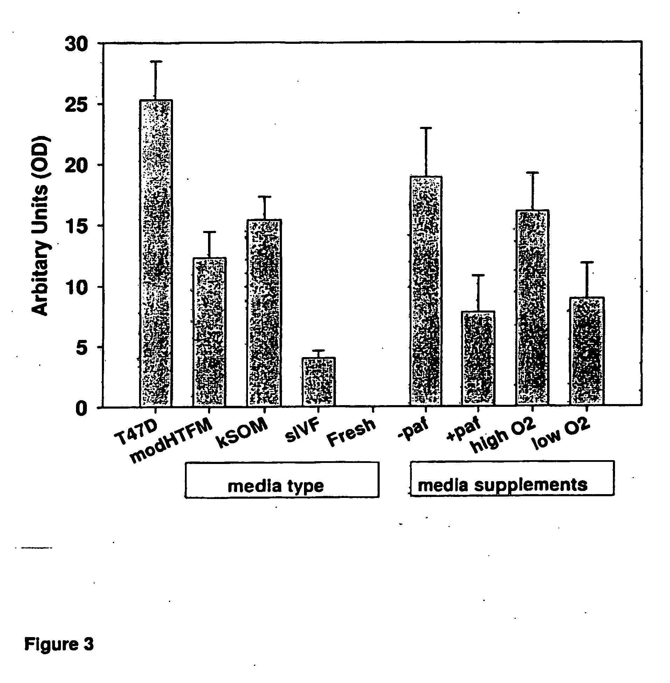

**Figure 3** provides a graph of the quantitative analysis of p53 protein expression within culture media in which embryos fertilized *in situ* and cultured to the blastocyst stage were cultured in different media formulations, or different media supplements. The media formulations were modified HTF; kSOM; or sIVF sequential culture media. C57BI embryos cultured in mod HTF were either deprived of platelet-activating factor (- paf), or had Paf added as a supplement (+ paf), or were cultured *in vitro* in either 20% O₂ (high O₂) or 5% O₂ (low O₂).

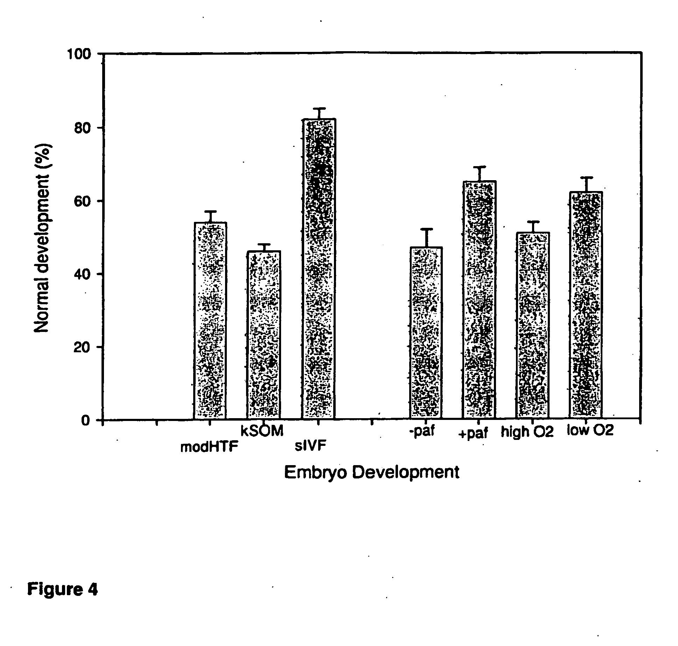

**Figure 4** provides a graph which correlates p53 release, as illustrated in Figure 3, and embryo development (% normal development) in media formulations or with media supplements as defined for Figure 3. Mouse zygotes were cultured *in vitro* for 96h and the proportion of embryos that developed to normal blastocysts were recorded.

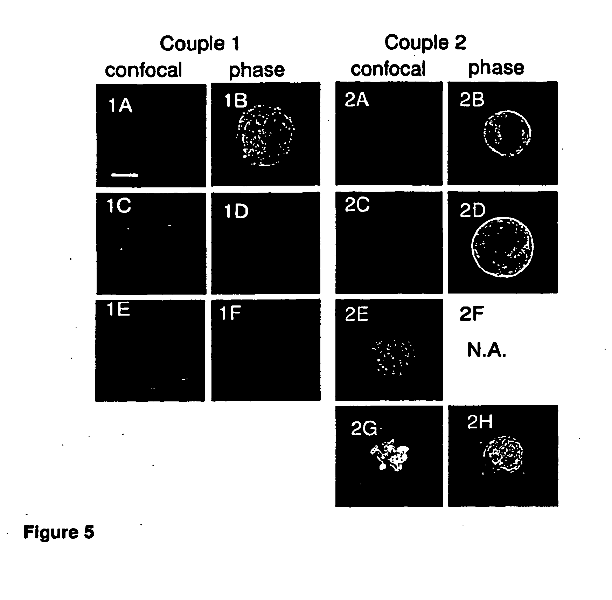

**Figure 5** provides photomicrographs of human embryos stained with an antibody to p53 and photographed using a confocal microscope. The micrographs show the pattern of p53 labeling in several embryos from 2 couples (1 & 2) with either non-immune IgG (1 A and 2A) or anti-p53 antibody (all other embryos). Two images of each embryo (except 2C) are provided: an equatorial (5µm) confocal section (confocal) or phase contrast (phase). All images were taken with identical microscope, laser settings and magnification.

### Abbreviations

- ART: assisted reproductive technologies
- BSA: bovine serum albumin
- BT: blastocyst transfer
- FITC: fluoresceine isothiocyanate
- FSH: follicle stimulating hormone
- GIFT: gamete intrafallopian transfer
- hCG: human chorionic gonadotrophin
- HTF: human tubal fluid
- ICSI: intracytoplasmic sperm injection
- IVF: *in vitro* fertilization
- kSOM: potassium modified simplex optimized media
- Paf: platelet activating factor
- PBS: phosphate buffered saline
- sIVF: Sydney IVF (brand name)
- ZIFT: zygote intrafallopian transfer

### Best Mode of Performing the Invention

The expression of p53 has been demonstrated to be upregulated in embryos produced by assisted reproductive technologies (ART) such as *in vitro* fertilization (IVF) and that this upregulation correlates with poor embryo viability following ART (WO 2005/019440). Further, short term inhibition of p53 during the culture of embryos *in vitro* can increase the viability of embryos. For example the proportion of embryos developing into morphologically normal blastocysts can be increased, the number of cells per blastocyst increased and the number of cells in the embryo undergoing apoptosis decreased.

p53 is a transcription factor whose actions are normally located within the nucleus of cells. It is not generally considered a secretion product of cells. It has now been found, however, that p53 produced by embryos, in particular those generated by ART, is released into the *in vitro* culture medium and that this p53 release is inversely correlated with the viability of the embryo.

The detection of p53 in fluids, for example in sputum and in pancreatic juice and plasma of patients with pancreatic carcinomas has been reported. However, the presence of p53 and the *p53* gene in these cases is due to the presence of exfoliated cells (see Gao *et al.,* 2005). p53 has not previously been reported as being released by cells, nor have there been any reports of using protein release by cells as a diagnostic or predictive indicator of embryo viability or developmental potential.

In the present study two further normally intracellular survival/stress related proteins, Cav_{1.2} (a voltage-dependent calcium ion channel) and catalase, have also been detected in embryo culture media.

Accordingly, in one aspect there is provided a method for determining the viability of an embryo, the method comprising:
(a) culturing an embryo or pre-embryonic cell mass *in vitro* in culture medium; and
(b) detecting and/or measuring the amount of at least one marker in the culture medium,
wherein the level of the at least one marker detected in the culture medium is indicative of the developmental potential or viability of the embryo.

The method for determining the viability of an embryo is of particular benefit in assessing embryo viability and developmental potential during ART, and in particular IVF. Other suitable ART techniques to which the method described herein is applicable include, but are not limited to, gamete intrafallopian transfer (GIFT), zygote intrafallopian transfer (ZIFT), blastocyst transfer (BT) and intracytoplasmic sperm injection (ICSI). Thus an application of the method is in the selection of the most suitable embryos for transfer during ART. However, those of skill in the art will appreciate that the advantages offered by the method are not limited to ART-generated embryos. Rather the methods for determining the viability of an embryo provided herein are equally applicable to assessing viability of any embryos, whether they are produced *in vitro* via ART or in the reproductive tract of the animal. The methods provided herein are therefore also applicable to assessing viability of embryos in otherwise unassisted pregnancies and thus the likelihood of the embryo developing to full term.

The methods and compositions provided herein are of use not only for human reproduction, but for a variety of species. For example the methods and compositions provided herein can be used to assess embryo viability in animal husbandry, for species of agricultural value, and in species bred for conservation purposes. In particular the present invention finds application in vertebrates, and more particularly in mammals. For example, while studies have identified that the expression of intracellular p53 is elevated in human embryos with retarded development or abnormalities, the present study has identified that the elevated intracellular levels of p53 are expressed in murine embryos having a similarly reduced viability.

Further, the methods and compositions provided herein also find applications in circumstances in which it is beneficial to determine embryo viability, for example in the production of embryonic stem cells, the production of cloned embryos and all related techniques.

As disclosed herein, in models where the levels of p53 expression is experimentally manipulated, the subsequent development of embryos to viable fetuses after embryo transfer was inversely related to their expression of p53 and the release of p53 into the culture medium; those conditions resulting in higher levels of p53 expression and release resulted in fewer embryos displaying normal development through to blastocyst stage. As demonstrated in WO 2005/019440, temporary inhibition of p53 in the developing embryo leads to a significant increase in the proportion of embryos developing normally to blastocyst stage (as well as an increase in the number of cells per blastocyst and a decrease in the number of cells in the embryo undergoing apoptosis). Therefore, it will be readily appreciated by those skilled in the art that the methods provided herein in combination with the methods and compositions for improving embryo viability as disclosed in WO 2005/019440 may provide powerful and valuable avenues for the screening and treatment of embryos in circumstances in which it is beneficial to improve embryo viability, for example in improving the success rates of ART, in producing embryonic stem cells and cloned embryos and in all related techniques.

The employment of methods provided herein may have further potential applications including in the quality control of embryo culture procedures and quality assurance of the manufacturing and procedural aspects of ART. The method provided herein may provide tools for the design and creation of a rational and effective means for improved embryo culture media design, laboratory equipment and procedures, risk assessment and quality assurance/ quality control procedures throughout the ART process. For instance, using the methods described herein to determine embryo viability animal embryos may be used to screen materials such as culture media or culture additives to determine whether they support or retard embryo viability *in vitro.* Such screening methods may be used, for example, in the development of new media for ART techniques, or in the batch screening of media for quality control purposes. Similarly, the methods described herein for identifying embryo viability following embryo culture may be used to develop new techniques and methods for embryo culture, or to monitor ongoing culture techniques for compliance to standards.

At present, quality control of routine ART laboratory procedures and media production is most commonly performed by the use of a mouse embryo toxicity test. In this assay a large number of embryos (∼100) are cultured from the zygote stage through to the blastocyst stage for each test parameter. Toxicity reduces the proportion of embryos that develop to the blastocyst stage. This assay successfully detects gross toxicity within culture systems, but has limited utility for detailed quality assurance, quality control or research and development due to the poor sensitivity and specificity of the assay. It is possible to improve the predictive power by performing embryo transfer procedures of these sentinel embryos to foster mothers, however this is prohibitively expensive, requires a further large number of animals as foster mothers, and requires a further 2-3 weeks before a result is available. The methods provided herein provide a more straightforward and rational alternative for quality control while avoiding or ameliorating the inherent disadvantages and problems associated with the mouse embryo toxicity test.

Included within the scope of the methods described herein is the detection and/or measurement of a one or more cellular markers released by the embryo or embryo precursor. Such a marker will be a polypeptide or oligopeptide fragment thereof, and may be a glycoprotein or a lipoprotein or oligopeptide fragment thereof, which is/are normally associated with cell or embryo survival and/or stress responses. By way of example only, markers may include p53, Ca_{v1.2} or catalase.

Accordingly, the detection of any one or more of these markers in culture medium from cultured embryos is contemplated in a method for determining the viability of an embryo.

As exemplified herein a, protein marker p53 in the culture medium may be detected and quantified using the method of Western blotting. Polyclonal antibodies to human and other species p53, for example, suitable for use in Western blots are available commercially, for example from Abcam (p53 antibody ab2433). However those skilled in the art will appreciate that the invention is not limited to the use of such a method. Any method, direct or indirect, that is able to detect protein or polypeptide presence and/or measure protein expression or concentration will be useful in determining the level of a protein or polypeptide and is contemplated as forming part of the present invention. For example, it will be apparent that ELISA kits for the detection of p53 are also commercially available (for example the p53 ELISA Kit, PathScan® from Cell Signaling Technology) and that these may be used in the quantification of p53 levels in culture medium either directly or following concentration of the p53 component in the culture medium.

Methods of the present invention may make use of one or more antibodies for the detection of protein and polypeptide markers and the determination of marker levels. The antibodies may be polyclonal or monoclonal and may be raised by the use of the protein marker or an antigenic fragment or portion thereof as an antigen. Antibody binding may be detected by virtue of a detectable label on the primary antibody. Alternatively, the primary antibody may be detected by virtue of its binding with a secondary antibody or reagent that is appropriately labeled to enable detection. A variety of methods are known in the art for detecting binding in an immunoassay and are within the scope of the present invention. For example determinations of protein and polypeptide marker levels can be accomplished by any one of a number of techniques known in the art including, for example enzyme-linked immunosorbent assays (ELISA); sandwich immunoassays, immunoradiometric assays (IRMA), radioimmunoassays (RIA), immunoelectrophoresis assays, *in situ* immunoassays, immunodiffusion assays, immunofluorescence assays, chromatographic assays, mass spectroscopy such as surface-enhanced laser desorption-ionisation time-of-flight mass spectrometry (SELDI-TOF MS), ligand-binding assays, and the like. Detection methods may be in the solid phase, such as chip-based assays for cost-effective and efficient testing of multiple samples. Where the level of the marker present in the culture medium is low, it will be understood that methods for the concentration of proteins or peptides in a sample, such as size exclusion ultracentrifugation concentration techniques, may be used to increase the level of the marker in order to increase the sensitivity of detection.

As contemplated herein, methods of the invention may include the step of comparing the level of the one or more markers in embryo culture medium with the level of the same one or more markers from a control sample. Typically the control sample may be: (1) media that is identical to the test media but not exposed to embryos; (2) a positive control media, that is, pooled media standards from embryos shown to have high developmental potential; (3) a negative control media, that is, pooled media standards from embryos shown to have poor developmental potential. These controls would be developed specifically for each species and indication for which the assay is used. When ranking embryos by viability, it is contemplated that embryos which release the least p53 into the culture medium, for example, are the most viable, and conversely, embryos which release the most p53 into the culture medium are the least viable.

Also provided herein are methods for the screening or identification of candidate agents useful for modulating embryo viability, the processes comprising contacting an embryo or embryo precursor with a candidate agent, culturing the embryo *in vitro* and determining whether the level of one or more markers in the culture medium is increased or decreased in the presence of the agent and thereby determining whether the agent is capable of modulating, i.e., increasing or decreasing embryo viability. Based on the observations described herein, should the presence of the candidate agent result in a reduction in the level of a marker such as p53 in the culture medium, the agent may be considered as an agent suitable for use in increasing embryo viability. The determination of whether the candidate agent increases or decreases the level of one or more markers may be made by comparing the level of the marker produced by an embryo exposed to the agent with the level of the marker produced by an embryo not exposed to the agent, or by reference to a standard curve which correlates embryo viability with the level of the marker.

Typically the candidate agents are compounds that are not previously known to inhibit the expression or activity of the one or more markers detected in the culture medium.

The present application also describes kits for the determination of the level of a marker indicative of the developmental potential or viability of the embryo in culture medium wherein the kits facilitate the employment of methods of the invention.

There is also described a kit for determining the viability of an embryo or embryo precursor, the kit comprising at least one agent for measuring the level of a marker in culture media, and instructions for use of the agent to measure the level of the marker and thereby determine the embryo viability.

There is further provided according to the invention an *in vitro* method for determining the viability of an embryo or an embryo precursor, the method comprising:
(a) measuring the level of a marker in culture medium which has been used to culture an embryo or an embryo precursor *in vitro*; and
(b) comparing the measured level of the marker in the culture medium to at least one level of the marker which is indicative of the viability of the embryo and thereby determining the embryo viability.

There is also provided according to the invention an *in vitro* method of ranking an embryo or embryo precursor on the basis of its viability, the method comprising:
(a) measuring the level of a marker in culture medium of each embryo or embryo precursor of a plurality of embryos or embryo precursors which have been cultured individually *in vitro* in culture medium; and
(b) comparing the measured level of the marker in the culture medium of each embryo to at least one level of the marker which is indicative of the viability of the embryo.

There is further provided an *in vitro* method of screening a candidate agent for the ability to modulate the viability of an embryo or embryo precursor, the method comprising measuring the level of a marker which is indicative of the viability of the embryo in culture medium of a first embryo or embryo precursor which has been contacted with the candidate agent and cultured *in vitro* in the culture medium, and in culture medium of a second embryo or embryo precursor which has been cultured *in vitro* in the culture medium but which has not been exposed to the candidate agent, comparing the measured level of the marker in the culture medium of the first and the second embryo or embryo precursor and thereby determining whether the candidate agent modulates embryo viability.

There is also provided an *in vitro* method of screening a candidate agent for the ability to modulate the viability of an embryo or embryo precursor, the method comprising measuring the level of a marker which is indicative of the viability of the embryo in culture medium of an embryo or embryo precursor which has been contacted with the candidate agent and cultured *in vitro* in the culture medium, comparing the measured level of the marker in the culture medium with at least one level which is indicative of the viability of the embryo and thereby determining whether the candidate agent modulates embryo viability.

There is also provided according to the invention *in vitro* use of an agent to detect or measure a marker in culture medium of a cultured embryo or embryo precursor, wherein the level of the marker is indicative of the viability of the embryo.

There is further provided *in vitro* use of an agent to measure the level of p53 in culture medium of a cultured embryo or embryo precursor.

The present invention will now be further described in greater detail by reference to the following specific examples, which should not be construed as in any way limiting the scope of the invention.

### Examples

### General Methods

*Mice and IVF:* The strain of mice used in experiments was C57BU6J. All animals were housed and bred in the Gore Hill Research Laboratory, St Leonards, NSW, Australia. All animals were under 12h light: 12 h dark cycle and had access to food and water *ad libitum.* Four to eight week old females were superovulated by i.p. injection of 10 IU pregnant mare serum gonadotrophin (Folligon, Intervet International, Boxmeer, The Netherlands) followed 48 h later by 10 IU human chorionic gonadotrophin (hCG, Chorulon, Intervet). Females were paired with males of proven fertility. Day 1 of pregnancy was confirmed by the presence of a copulation plug the following morning.

*Embryo collection and culture:* Mice were killed by cervical dislocation. Cumulus masses or embryos were flushed from the reproductive tract with HEPES-buffered modified human tubal fluid medium (HEPES-modHTF - 101.6 mM NaCl, 4.6 mM KCI, 0.2 mM MgSO₄, 0.4 mM KH₂PO₄, 21.4 mM Na lactate, 1 mM glutamine, 2 mM CaCl₂, 4 mM NaHCO₃, 0.33 mM Na pyruvate, 2.78 mM glucose and 21 mM HEPES buffer pH 7.35; 285 mOsm/l). All components of the media were tissue culture grade (Sigma Chemical Company, St Louis, MO) and contained 3 mg BSA /ml unless otherwise stated (CSL Ltd., Melbourne, Vic., Australia).

Zygotes were collected 20-21 h after hCG and freed from their cumulus cells by brief exposure to 300 IU hyaluronidase (Sigma) in HEPES-HTF. Embryos were thoroughly washed in three changes of Hepes-modHTF, recovered in minimal volume and assigned to various treatments as required in mod-HTF (same as Hepes-mod-HTF except that Hepes buffer was replaced with NaHCO₃). Embryos were cultured in 10 µl volumes in 60-well HLA plates (LUX 5260, Nunc, Naperville, IL) overlaid by approximately 2 mm of heavy paraffin oil (Sigma). Plates were allowed to equilibrate in the culture incubator for at least 4 h before addition of the embryos. Embryos were cultured individually for 96 h at 37°C in 5% CO₂. Fresh 2-cell embryos were collected on 40-42 h after hCG.

### Example 1

### Immunolocalisation of p53 in mouse and human embryos

The expression pattern of p53 protein in pre-implantation mouse embryos was investigated using immunofluorescence.

Embryos were washed 3-times (washing media: PBS with 0.1 % BSA, 0.1 % Tween-20) and fixed with fresh 2% paraformaldehyde (Sigma) in PBS PH 7.4 for 1h at room temperature. Embryos were permeabilised for 30 min at room temperature in PBS with 2% BSA, 0.2% Tween-20 and 0.2% Triton X-100 and blocked in 2% BSA and 30% blocking serum for 1-3h. P53 antigen was stained overnight at 4°C with 2 µg anti-p53 sheep polyclonal antibody /ml (Oncogene Research Products (PC35) or rabbit polyclonal anti-Bax (Santa-Cruz, N-20) in PBS with 2% BSA and then 5 µg rabbit anti-sheep FITC-conjugated antibody/ml for 1h at room temperature (green channel). Controls were 2 µg non-immune IgG/ml instead of primary antibody. Immunofluorescence images were observed using a BioRad Radiance confocal microscope (Australian Key Centre for Microscopy and Microanalysis, University of Sydney) using a Nikon Plan Apo 60X/1.40 oil immersion objective. Images were captured using LaserSharp 2000 Version 4.0 (build 365) software. Microscope and laser settings were adjusted such that no fluorescence was observed with non-immune control. All test specimens were observed with these settings and settings the same. Quantitative analysis of staining was performed using NIH Image software. For imaging the whole embryo 1 µM sections were performed using the z-sectioning facility of the microscope.

The expression of immunodetectable p53 protein was low in preimplantation mouse embryos collected fresh from the reproductive tract (fresh embryos) (Figure 1A). Embryos produced by IVF and cultured individually in 10 µL of media (Figure 1B) or those fertilized in the reproductive tract and then cultured *in vitro* in groups of 10 in 10 µL of media (Figure 1C) showed different patterns of p53 expression. There was little immunodetectable p53 at the morula through blastocyst stage in fresh embryos and no evidence of accumulation of protein within the nucleus of the embryos cells. In contrast, there was a substantial accumulation of p53 in IVF embryos with intense nuclear localization (Figure 1C). There was only modest difference in p53 prior to the 8-cell stage, but greatly increased expression at each stage thereafter (data not shown). By the 8-cell stage onwards, many IVF embryos showed nuclear staining as the predominant pattern of staining with relatively less cytoplasmic staining. This pattern of protein expression correlates with the pattern of embryopathy after ART, with most embryo loss occurring after the 8-cell stage.

A total of 8 human embryos from two couples were examined. Embryos were created by standard IVF as previously described in detail in Henman *et al* (2005). Embryos were those excess to use by the treated couples and were donated with informed consent under NHMRC licence 309702B.

Gametes were fertilized in Sydney IVF Fertilization media, and then cultured in Sydney IVF Cleavage media (48h) followed by cultured in Sydney IVF Blastocyst media (all media from Cook). Following culture for this period embryos were frozen using a Sydney IVF Blastocyst Freezing Kit (Cook). Following culture individual embryo were placed in straws (IMV Technologies, France) and loaded into a rate-controlled freezer (Kryo 10 series II; Planar, Middelsex, England). Straws were cooled to -7°C at 2°/min, then -7°C to -30°C at 0.3°/min, and from -30°C to -150°C at 35°/min, then plunged directly into liquid nitrogen at -196°C.

Blastocysts were thawed by removing the straw from the liquid nitrogen and exposing the straw to room temperature for 30 s, followed by 30 s in a 30°C water bath. The straw was cut open onto a small dry petri dish and the contents moved promptly into the first of a four-stage thawing process (Sydney IVF Blastocyst Thawing Kit; Cook). Embryos were immediately fixed in formaldehyde and subjected to immuno-labelling for P53 as is described for mouse embryos.

Immunofluorescence procedures were the same for both human and mouse embryos. Embryos were washed 3 times in phosphate buffered saline (PBS) with 0.1% (w/v) bovine serum albumin, 0.1% (v/v) Tween-20 and 0.2% (w/v) sodium azide (washing buffer) and then fixed with freshly prepared 2% paraformaldehyde (w/v) (Sigma) in PBS (pH 7.4) for 30 min and then permeabilized with 2% paraformaldehyde with 0.3% Tween-20 (Sigma) at room temperature for a 30 min. Embryos were washed 3 times in washing solution and then were blocked in PBS containing 2% (w/v) BSA, and 30% (v/v) goat serum for 3h. They were stained overnight at 40C with primary antibodies [1:500 anti-p53 (Ab-7) polyclonal antibody (Cat No: PC35, Oncogene Research Products) or an equivalent concentration of isotype control immunoglobulin (negative control). Primary antibodies were detected by incubation of embryos with secondary antibody coupled to FITC in PBS, 2% BSA for 1 h at room temperature. Optical sectioning was performed with a Bio-Rad Radiance Confocal microscope, using a Nikon Plan Apo 60X/1.4 oil emersion objective. Images were captured using Lasersharp 2000, Version 4.0 (BioRad). Microscope and laser settings were adjusted such that no fluorescence was observed with non-immune controls. All the test specimens were observed with these same settings.

Staining of the 8 human embryos for immuno-detectable p53 showed considerable within and between embryo heterogeneity of p53 expression. Four embryos with the morphology of normal blastocysts displayed generally low levels of p53 staining, but in some embryos showed some cells with clear p53 expression. One embryo showing retarded development and abnormal morphology displayed uniformly high levels of p53 expression in cells in an equatorial section. In a blastocyst that had a partially collapsed blastocoel, there were a large number of cells that showed p53 staining. Much of this staining appeared to be associated with the nuclear region of cells.

Expression of p53 was also identified in the zona pellucida and peri-viteline space, which is consistent with the observation in the mouse that under conditions of high p53 expression some of the p53 is released by the embryo.

This study shows that some embryos produced by ART manifest a high rate of p53 protein expression. The highest levels of p53 expression occurred in embryos with poor morphology.

### Example 2

### p53 expression in mouse embryos

Western blot analysis was used to compare the expression of p53 protein in mouse embryos fertilized and grown in the reproductive tract without further culture and in IVF mouse embryos.

Two males 10-20 wk of age and of proven fertility were used for each IVF procedure. Following cervical dislocation, the epididymides were removed and placed into 1 mL of pre-equilibrated HTF in a petri dish. The epididymides were punctured with a sterile needle and the sperm gently squeezed out into medium. The dish was placed into an incubator at 37°C with 5% CO₂ in air for 40 min to allow sperm to disperse. Cumulus masses containing oocytes were then collected from female mice 15-17 h after the hCG injection and extensively washed in Hepes-HTF. Groups of approximately 30 oocytes with their associated cumulus masses were placed into 5 mL plastic tubes (Falcon; Becton Dickinson Labware, Lincoln Park, NJ) in 1 mL of HTF. After dispersal of the sperm, they were thoroughly mixed and the concentration was assessed using a hemocytometer. Motile sperm (0.5 x 10⁶) were added to each tube containing oocytes.

The fertilization rate was assessed at 5-6 h after insemination by visualization of pronuclei. All fertilized oocytes were extensively washed in Hepes-HTF to remove sperm and cumulus cells and then pooled. Embryos of all types were recovered in a minimal volume and assigned to various treatments as required.

Cells of the T47D breast cell line (Cat # HTB-133, American Type Culture Collection, Manassas, VA USA) was used as a positive control for the detection of p53.

Embryos were collected and washed 3 times in cold PBS and transferred in a maximum volume of 1.5 µl PBS. Embryo culture media aliquots had embryos and all cells removed and then subjected to freeze drying. The lyophilized proteins were redissolved in 1.5 µl of water. Embryo or reconstituted culture media was mixed with 1.5 µl of 2X extraction buffer (2X PBS, 2% Triton X-100, 24 mM deoxycholic acid, 0.2% sodium dodecyl sulfate, 20 mM NaF, 20 mM Na₄P₂O₇, 2 mM PMSF, 3.08 µM Aprotinin, 42µM Leupeptin and 2.91 µM Pepstatin A- all from Sigma). The embryos were lysed by three cycles of freezing in liquid nitrogen and thawing (with vortexing).

Protein samples were diluted with 1 µl of 5X Laemmli buffer (50 mM Tris-HCl, 5 mM EDTA pH 8.0, 12.5% Sodium dodecyl sulfate, 0.05% bromophenol blue and 25% beta-Mercaptoethanol), incubated 10 min at 60°C and ran on 20% homogenous SDS polyacrylamide gels (Pharmacia) using PhastSystem apparatus (PhastSystem- separation and control unit, Pharmacia, Sweden).

Proteins were blotted into PVDF membranes (Hybond-P, Amersham Pharmacia) in a semi-dry blotting apparatus overnight using transfer buffer (12 mM Tris PH 7.0, 96 mM Glycine and 20% methanol). Nonspecific binding was blocked by 5% skim milk in PBS supplemented with 0.05% Tween-20 (PBST) at room temperature for 1 h. Membranes were probed with 1:500 diluted anti P53 antibody overnight at 4°C in 2.5% skim milk, and detected with 1:500 diluted horse radish peroxidase (Jackson ImmunoResearch Laboratories, West Grove, PA, USA) conjugated mouse antibody. Membranes were developed with Pico SuperSignal Chemiluminescent Substrate (Pierce, Rockford, IL, USA) for 5 min at room temperature.

The results of these experiments are illustrated in Figure 1D. Figure 1D clearly demonstrates that p53 expression in the embryo increases with development of the embryo, at least from a 1-cell embryo through to blastocyst. Further, p53 expression is higher in embryos cultured *in vitro* than in embryos collected fresh from the reproductive tract. The increased p53 expression observed following *in vitro* culture was not a consequence of an overall change in the pattern of protein expression in the embryos, as evidenced by the maintenance of expression of the constitutively expressed protein LIS-1.

### Example 3

### Detection of p53 in embryo culture media

Western blot analysis was used to determine the concentration of p53 protein within culture media resulting from the culture of mouse blastocysts produced by IVF, fertilised *in situ* and then cultured to blastocyst stage *in vitro* (ISF), or blastocysts collected fresh from the reproductive tract (fresh) and placed in media for 6 hours.

Embryos were collected from mice housed and bred in the Gore Hill Research Laboratories, St. Leonards, NSW, Australia, under a 12 hour light: 12 hour dark cycle and had access to food and water ad libitum. Four to eight-wk-old females were superovulated by intraperitoneal injection of 10 IU of eCG (Folligon; Intervet International, Boxmeer, the Netherlands) followed 48 h later by 10 IU of hCG (Chorulon; Intervet International).

For in situ fertilization (ISF) techniques embryos fertilization was by natural mating. Females were paired with males of proven fertility. Pregnancy was confirmed by the presence of a copulation plug the following morning (Day 0.5). ISF zygotes were flushed from the reproductive tract with Hepes-buffered modified human tubal fluid medium (Hepes-HTF) and were cultured in modified HTF medium (mod-HTF).

IVF was performed by collecting sperm from two males, 10-20 wk of age, and of proven fertility. Following cervical dislocation, the epididymides were removed and placed into 1 mL of pre-equilibrated HTF in a petri dish. The epididymides were punctured with a sterile needle and the sperm gently squeezed out into medium. The dish was placed into an incubator at 37C with 5% CO2 in air for 40 min to allow sperm to disperse. Cumulus masses containing oocytes were then collected 15-17 h after the hCG injection and extensively washed in Hepes-HTF. Groups of approximately 30 oocytes with their associated cumulus masses were placed into 5-ml plastic tubes (Falcon; Becton Dickinson Labware, Lincoln Park, NJ) in 1 mL of HTF. After dispersal of the sperm, they were thoroughly mixed and the concentration was assessed using a hemocytometer. Motile sperm (0.5 x 10⁶) were added to each tube containing oocytes. The fertilization rate was assessed at 5-6 h after insemination by visualization of pronuclei. All fertilized oocytes were extensively washed in Hepes-HTF to remove sperm and cumulus cells and then pooled.

All components of the media were tissue culture grade (Sigma Chemical Company, St. Louis, MO) and contained 3 mg/ml of BSA unless otherwise stated (CSL Ltd., Melbourne, Victoria, Australia). Zygotes were collected 20-21 h after hCG injection and were freed from their cumulus cells by brief exposure to 300 IU of hyaluronidase (Sigma Chemical Company) in Hepes-HTF. Embryos were recovered in minimal volume and were assigned to various treatments as required in mod-HTF.

Embryos were cultured in 10µl volumes in 60-well human leukocyte antigen plates (LUX 5260; Nunc Inc., Naperville, IL) overlaid by approximately 2 mm of heavy paraffin oil (Sigma Chemical Company). Embryos were cultured in groups of 10. Culture was at 37°C in 5% CO₂ for the periods indicated in individual experiments.

Embryos were prepared by IVF or collected at the zygote stage (ISF) (Day 0.5) and were cultured in vitro for 90 h (cultured) or were collected directly from the uterus at the blastocyst stage (Day 3.5).

Media conditioned by 3,000 T47D cancer cells were used as a control.

As shown in Figure 2, no extracellular p53 protein was detected in culture medium in which fresh blastocysts were placed. Similarly, there was little detectable p53 in ISF blastocysts. In contrast, a significant amount of p53 detected in IVF blastocysts.

Thus, this experiment demonstrates that p53 is released by cultured embryos into the culture medium, and that in IVF embryos which are characterized by poor viability the amount of p53 which is released is elevated compared to embryos which are characterized by good viability.

### Example 4

### Correlation of p53 concentration in culture media with embryo developmental potential

As illustrated in Figure 3, a quantitative analysis was conducted of p53 protein expression within culture media in which embryos fertilized *in situ* and cultured to the blastocyst stage were cultured in different media formulations, or different media supplements. Embryos were cultured in several types of media, containing in all cases 3mg bovine serum albumin/ml. Media was either mod HTF (as described in O'Neill, 1997) kSOM (as described in Lawitts, & Biggers, 1993) or sIVF sequential media (sIVF cleavage media for 48h and then sIVF blastocyst media for 48h - as supplied by Cook Australia Pty Ltd).

In some experiments modHTF was supplemented with Paf (Sigma). An equal mixture of 1-o-octadecyl/hexadecyl-2-acetyl-sn-glycero-3-phosphocholine (Paf) was prepared as a 1mg/ml stock solution in chloroform. Aliquots were removed to a siliconized glass test tube, reduced to dryness under a steam of N₂ and dissolved in perfusion medium to the desired concentration. Culture was performed in a final concentration of 0.1 µM Paf.

Culture was performed normally under an atmosphere containing 20% oxygen except where indicated as low O₂, where a mix of 5% O₂, 5% CO₂ in N₂ was applied.

p53 was detected by Western blot as described above and densitometry was performed using Scion Image software (Scion Corporation, Frederick, Maryland, USA).

As illustrated in Figure 3, the extra-embryonic concentration of p53 was found to be higher in modified HTF and kSOM media, and when embryos were cultured in the absence of Paf or under high (20%) O₂ conditions.

The level of p53 release into culture media as illustrated in Figure 3 was subsequently determined to be closely inversely correlated with embryo developmental potential. The developmental stage and morphology of embryos was assessed by visualizing the embryos with an inverted phase contrasted microscope (Nikon Diaphot, Japan) at 24 h interval after zygote collection. After 96 h culture cell counts and integrity of nuclei were assessed by visualization of cell nuclei following staining with 4 µg Hoechst dye 33342/ml (Sigma). Embryos were left in this solution for 40 min and then prepared as wet mounts on a glass microscope slide under coverslip. Nuclei were visualized using mercury lamp UV illumination and epiflourescence on a Nikon Optiphot microscope with an Olympus DPlan Apo 40 UV objective. Individual nuclear morphology was categorized as normal (round/ovoid/mitotic) or abnormal (punctuate/pyknotic/fragmented).

Figure 4 illustrates the percentage of mouse zygotes that developed normally to blastocyst stage following 96h in vitro culturing under the conditions (culture media type and media supplementation) as shown in Figure 3. It can be seen that under those conditions in which p53 release into the media was higher, the percentage of zygotes developing normally to blastocysts was least.

### Example 5

### Confirmation of embryo viability

Embryo viability detected using the detection of a marker in the embryo culture medium is assessed by determining the rate of pregnancy outcomes using a modification of the techniques described by Roudebush *et al.* (2002).

Embryos generated for IVF are individually assessed for their ability to secrete p53 into the culture medium, as described in previous examples. Embryos thus assessed are transferred into subjects using standard IVF procedures, and the rate of pregnancy which results is correlated with the level of expression of p53 released by the embryo into the culture medium.

Using the data generated by such a study, a threshold level of marker in the culture medium may be selected which will predict an embryo's viability which will produce a desired rate of resultant pregnancy.

Also provided are kits for the determination of the level of a marker indicative of the developmental potential or viability of the embryo in culture medium, wherein the kits facilitate the employment of methods of the invention. Typically, kits for carrying out a method of the invention contain all the necessary reagents to carry out the method. The kit may comprise a first container containing an antibody raised against p53 and a second container containing a conjugate comprising a binding partner of the antibody, together with a detectable label.

Typically, the kits described above will also comprise one or more other containers, containing for example, wash reagents, and/or other reagents capable of quantitatively detecting the presence of bound antibodies. Preferably, the detection reagents include labeled (secondary) antibodies or, where the antibody raised against p53, or Ca_{v1.2} or catalase is itself labeled, the compartments comprise antibody binding reagents capable of reacting with the labeled antibody. In the context of the present application, a compartmentalized kit includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept the test sample, a container which contains the antibody(s) used in the assay, containers which contain wash reagents (such as phosphate buffered saline, Tris-buffers, and like), and containers which contain the detection reagent.

Typically, a kit may also include instructions for using the kit components to conduct the appropriate methods.

Methods of the present invention and the kits described may be equally applicable to any animal, including humans, for example including non-human primate, equine, bovine, ovine, caprine, leporine, avian, feline, canine and murine species. Accordingly, for application to different species, a single kit may be applicable, or alternatively different kits, for example containing reagents specific for each individual species, may be required.

### References

Bowman, P., and A. McLaren, Journal of Embryology and Experimental Morphology 23, 693-704 (1970).
Brison, D.R. et al., Hum. Reprod. 19, 2319-2324 (October 1, 2004, 2004).
Bryant, J., et al., AIHW Cat. No. PER 26. Sydney: Australian Institute of Health and Welfare National Perinatal Statistics Unit (Assisted Reproductive Technology Series No. 8). (2004).
Henman M, et al. Fertil Steril ;84:1620-7 (2005)
Lawitts and Biggers, Methods Enzymol. 225, 153-164 (1993).
O'Neill, C., Biology of Reproduction 56, 229-237 (1997).
O'Neill, C., Biology of Reproduction 58, 1303-1309 (1998).
O'Neill, C., Human Reproduction Update 11, 215-28 (2005).
Roedebush WE et al. Human Reprod. 17, 1306-1310 (2002).
Turner, K., et al., Human Reprod. 9, 2362-2366 (1994).

## Claims

1. A method for determining the viability of an embryo or an embryo precursor, the method comprising:
(a) culturing an embryo or an embryo precursor *in vitro* in culture medium, wherein the embryo precursor is a fertilized egg, a zygote or a pre-embryonic entity which follows fertilization of an egg;
(b) measuring the level of a marker in the culture medium; and
(c) comparing the measured level of the marker in the culture medium to at least one level of the marker which is indicative of the viability of the embryo and thereby determining the embryo viability,
wherein the marker is p53.

2. The method of Claim 1, wherein the embryo or embryo precursor is produced by assisted reproductive technology.

3. The method of claim 2, wherein the assisted reproductive technology is *in vitro* fertilization.

4. A method of ranking an embryo or embryo precursor on the basis of its viability, the method comprising
(a) culturing individually a plurality of embryos or embryo precursors *in vitro* in culture medium, wherein the embryo precursor is a fertilized egg, a zygote or a pre-embryonic entity which follows fertilization of an egg;
(b) measuring the level of a marker in the culture medium of each embryo or embryo precursor;
(c) comparing the measured level of the marker in the culture medium of each embryo to at least one level of the marker which is indicative of the viability of the embryo,
wherein the marker is p53.

5. The method of claim 4, comprising the step of identifying the embryo with the greatest viability.

6. The method of Claim 1 or Claim 4, wherein the step (c) of comparing the measured level of the marker in the culture medium to at least one level of the marker which is indicative of the viability of the embryo comprises comparing the level of the marker in culture medium with the level of the marker from a control sample.

7. The method of Claim 6, wherein the control sample is a positive control comprising pooled culture media from embryos having high viability.

8. The method of Claim 8, wherein the control sample is a negative control comprising pooled culture media from embryos having low viability.

9. The method of Claim 1 or 4, wherein the step (c) of comparing the measured level of the marker in the culture medium to at least one level of the marker which is indicative of the viability of the embryo comprises comparing the level of the marker in the culture medium to the level of the marker in two or more control samples.

10. Use of at least one agent for measuring the level of a marker in culture media, wherein the marker is p53, for determining the viability of an embryo or embryo precursor, wherein the embryo precursor is a fertilized egg, a zygote or a pre-embryonic entity which follows fertilization of an egg, wherein the at least one agent comprises an antibody to p53.

11. A method of screening a candidate agent for the ability to modulate the viability of an embryo or embryo precursor, wherein the embryo precursor is a fertilized egg, a zygote or a pre-embryonic entity which follows fertilization of an egg, the method comprising contacting a first non-human embryo or non-human embryo precursor with the candidate agent, culturing the first embryo or embryo precursor in vitro in culture medium, culturing a second non-human embryo or non-human embryo precursor which has not been exposed to the candidate agent in vitro in culture medium, measuring the level of a marker which is indicative of the viability of the embryo in the culture medium of the first and the second embryo or embryo precursor, comparing the measured level of the marker in the culture medium of the first and the second embryo or embryo precursor and thereby determining whether the candidate agent modulates embryo viability, wherein the marker is p53.

12. A method of screening a candidate agent for the ability to modulate the viability of an embryo or embryo precursor, wherein the embryo precursor is a fertilized egg, a zygote or a pre-embryonic entity which follows fertilization of an egg, the method comprising contacting a non-human embryo or non-human embryo precursor with the candidate agent, culturing the embryo or embryo precursor in vitro in culture medium, measuring the level of a marker which is indicative of the viability of the embryo in the culture medium, comparing the measured level of the marker in the culture medium with at least one level which is indicative of the viability of the embryo and thereby determining whether the candidate agent modulates embryo viability, wherein the marker is p53.

13. The method of claim 11 or claim 12, wherein the candidate agent is a culture medium.

14. The method of claim 11 or claim 12, wherein the modulation of embryo viability is an increase in embryo viability.

15. The method of any one of claim 1, claim 4, claim 11 or claim 12, wherein the embryo or embryo precursor is an embryo or embryo precursor of a non-human primate, equine, bovine, ovine, caprine, leporine, avian, feline, canine or a murine species.

16. The method of claim 1, or claim 4, wherein the embryo is a human embryo.

## Patentansprüche

1. Verfahren zum Bestimmen der Lebensfähigkeit eines Embryos oder eines Embryovorläufers, wobei das Verfahren die folgenden Schritte beinhaltet:
(a) Kultivieren eines Embryos oder eines Embryovorläufers *in vitro* in Kulturmedium, wobei der Embryovorläufer ein befruchtetes Ei, eine Zygote oder eine präembryonale Entität ist, die auf die Befruchtung eines Eies folgt;
(b) Messen des Anteils eines Markers in dem Kulturmedium; und
(c) Vergleichen des gemessenen Anteils des Markers in dem Kulturmedium mit wenigstens einem Anteil des Markers, der die Lebensfähigkeit des Embryos anzeigt, und dadurch Bestimmen der Lebensfähigkeit des Embryos,
wobei der Marker p53 ist.

2. Verfahren nach Anspruch 1, wobei der Embryo oder Embryovorläufer durch assistierte Reproduktionstechnik produziert wird.

3. Verfahren nach Anspruch 2, wobei die assistierte Reproduktionstechnik *In-vitro*-Befruchtung ist.

4. Verfahren zum Einstufen eines Embryos oder Embryovorläufers anhand seiner Lebensfähigkeit, wobei das Verfahren die folgenden Schritte beinhaltet:
(a) individuelles Kultivieren mehrerer Embryos oder Embryovorläufer *in vitro* in Kulturmedium, wobei der Embryovorläufer ein befruchtetes Ei, eine Zygote oder eine präembryonale Entität ist, die auf die Befruchtung eines Eies folgt;
(b) Messen des Anteils eines Markers in dem Kulturmedium von jedem Embryo oder Embryovorläufer;
(c) Vergleichen des gemessenen Anteils des Markers in dem Kulturmedium von jedem Embryo mit wenigstens einem Anteil des Markers, der die Lebensfähigkeit des Embryos anzeigt,
wobei der Marker p53 ist.

5. Verfahren nach Anspruch 4, das den Schritt des Identifizierens des Embryos mit der größten Lebensfähigkeit beinhaltet.

6. Verfahren nach Anspruch 1 oder Anspruch 4, wobei der Schritt (c) des Vergleichens des gemessenen Anteils des Markers im Kulturmedium mit wenigstens einem Anteil des Markers, der die Lebensfähigkeit des Embryos anzeigt, das Vergleichen des Anteils des Markers in dem Kulturmedium mit dem Anteil des Markers von einer Kontrollprobe umfasst.

7. Verfahren nach Anspruch 6, wobei die Kontrollprobe eine positive Kontrolle ist, die gepoolte Kulturmedien von Embryos mit hoher Lebensfähigkeit umfasst.

8. Verfahren nach Anspruch 6, wobei die Kontrollprobe eine negative Kontrolle ist, die gepoolte Kulturmedien von Embryos mit geringer Lebensfähigkeit umfasst.

9. Verfahren nach Anspruch 1 oder 4, wobei Schritt (c) des Vergleichens des gemessenen Anteils des Markers in dem Kulturmedium mit wenigstens einem Anteil des Markers, der die Lebensfähigkeit des Embryos anzeigt, das Vergleichen des Anteils des Markers im Kulturmedium mit dem Anteil des Markers in zwei oder mehr Kontrollproben umfasst.

10. Verwenden von wenigstens einem Mittel zum Messen des Anteils eines Markers in Kulturmedium, wobei der Marker p53 ist, um die Lebensfähigkeit eines Embryos oder Embryovorläufers zu bestimmen, wobei der Embryovorläufer ein befruchtetes Ei, eine Zygote oder eine präembryonale Entität ist, die auf die Befruchtung eines Eies folgt, wobei das wenigstens eine Mittel einen Antikörper gegen p53 umfasst.

11. Verfahren zum Screenen eines in Frage kommenden Mittels im Hinblick auf die Fähigkeit zur Modulation der Lebensfähigkeit eines Embryos oder Embryovorläufers, wobei der Embryovorläufer ein befruchtetes Ei, eine Zygote oder eine präembryonale Entität ist, die auf die Befruchtung eines Eies folgt, wobei das Verfahren die folgenden Schritte beinhaltet: Inkontaktbringen eines ersten nicht humanen Embryos oder nicht humanen Embryovorläufers mit dem in Frage kommenden Mittel, Kultivieren des ersten Embryos oder Embryovorläufers in vitro in Kulturmedium, Kultivieren eines zweiten nicht humanen Embryos oder nicht humanen Embryovorläufers, der dem in Frage kommenden Mittel nicht ausgesetzt wurde, in vitro in Kulturmedium, Messen des Anteils eines Markers, der die Lebensfähigkeit des Embryos anzeigt, im Kulturmedium des ersten und des zweiten Embryos oder Embryovorläufers, Vergleichen des gemessenen Anteils des Markers im Kulturmedium des ersten und des zweiten Embryos oder Embryovorläufers und dadurch Bestimmen, ob das in Frage kommende Mittel die Embryolebensfähigkeit moduliert, wobei der Marker p53 ist.

12. Verfahren zum Screenen eines in Frage kommenden Mittels im Hinblick auf die Fähigkeit zur Modulation der Lebensfähigkeit eines Embryos oder Embryovorläufers, wobei der Embryovorläufer ein befruchtetes Ei, eine Zygote oder eine präembryonale Entität ist, die auf die Befruchtung eines Eies folgt, wobei das Verfahren die folgenden Schritte beinhaltet: Inkontaktbringen eines nicht humanen Embryos oder nicht humanen Embryovorläufers mit dem in Frage kommenden Mittel, Kultivieren des Embryos oder Embryovorläufers in vitro in Kulturmedium, Messen des Anteils eines Markers, der die Lebensfähigkeit des Embryos anzeigt, im Kulturmedium, Vergleichen des gemessenen Anteils des Markers im Kulturmedium mit wenigstens einem Anteil, der die Lebensfähigkeit des Embryos anzeigt, und dadurch Bestimmen, ob das in Frage kommende Mittel die Embryolebensfähigkeit moduliert, wobei der Marker p53 ist.

13. Verfahren nach Anspruch 11 oder Anspruch 12, wobei das in Frage kommende Mittel ein Kulturmedium ist.

14. Verfahren nach Anspruch 11 oder Anspruch 12, wobei die Modulation der Embryolebensfähigkeit eine Zunahme der Embryolebensfähigkeit ist.

15. Verfahren nach Anspruch 1, Anspruch 4, Anspruch 11 oder Anspruch 12, wobei der Embryo oder Embryovorläufer ein Embryo oder Embryovorläufer einer nicht humanen Primaten-, Pferde-, Rinder-, Schaf-, Ziegen-, Hasen-, Vogel-, Katzen-, Hunde- oder Maus-Spezies ist.

16. Verfahren nach Anspruch 1 oder Anspruch 4, wobei der Embryo ein humaner Embryo ist.

## Revendications

1. Méthode pour déterminer la viabilité d'un embryon ou d'un précurseur d'embryon, ladite méthode comprenant les étapes qui consistent à :
(a) cultiver un embryon ou un précurseur d'embryon *in vitro* dans un milieu de culture, dans laquelle le précurseur d'embryon est un oeuf fécondé, un zygote ou une entité pré-embryonnaire qui suit la fécondation d'un oeuf ;
(b) mesurer le taux d'un marqueur dans le milieu de culture ; et
(c) comparer le taux mesuré du marqueur dans le milieu de culture avec au moins un taux du marqueur qui est indicatif de la viabilité de l'embryon et déterminer ainsi la viabilité de l'embryon,
dans laquelle le marqueur est p53.

2. Méthode selon la revendication 1, dans laquelle l'embryon ou le précurseur d'embryon est produit par procréation médicalement assistée.

3. Méthode selon la revendication 2, dans laquelle la procréation médicalement assistée est la fécondation *in vitro.*

4. Méthode de classification d'un embryon ou d'un précurseur d'embryon en fonction de sa viabilité, la méthode comprenant les étapes qui consistent à :
(a) cultiver individuellement une pluralité d'embryons ou de précurseurs d'embryon *in vitro* dans un milieu de culture, dans laquelle le précurseur d'embryon est un oeuf fécondé, un zygote ou une entité pré-embryonnaire qui suit la fécondation d'un oeuf ;
(b) mesurer le taux d'un marqueur dans le milieu de culture de chaque embryon ou précurseur d'embryon ;
(c) comparer le taux mesuré du marqueur dans le milieu de culture de chaque embryon avec au moins un taux du marqueur qui est indicatif de la viabilité de l'embryon,
dans laquelle le marqueur est p53.

5. Méthode selon la revendication 4, comprenant l'étape qui consiste à identifier l'embryon présentant la plus forte viabilité.

6. Méthode selon la revendication 1 ou 4, dans laquelle l'étape (c) consistant à comparer le taux mesuré du marqueur dans le milieu de culture avec au moins un taux du marqueur qui est indicatif de la viabilité de l'embryon comprend la comparaison du taux du marqueur dans le milieu de culture avec le taux du marqueur obtenu dans un échantillon témoin.

7. Méthode selon la revendication 6, dans laquelle l'échantillon témoin est un témoin positif comprenant les milieux de culture regroupés provenant des embryons ayant une forte viabilité.

8. Méthode selon la revendication 6, dans laquelle l'échantillon témoin est un témoin négatif comprenant les milieux de culture regroupés provenant des embryons ayant une faible viabilité.

9. Méthode selon la revendication 1 ou 4, dans laquelle l'étape (c) consistant à comparer le taux mesuré du marqueur dans le milieu de culture avec au moins un taux du marqueur qui est indicatif de la viabilité de l'embryon comprend la comparaison du taux du marqueur dans le milieu de culture avec le taux du marqueur dans deux ou plusieurs échantillons témoins.

10. Utilisation d'au moins un agent pour mesurer le taux d'un marqueur dans les milieux de culture, dans laquelle le marqueur est p53, pour déterminer la viabilité d'un embryon ou d'un précurseur d'embryon, dans laquelle le précurseur d'embryon est un oeuf fécondé, un zygote ou une entité pré-embryonnaire qui suit la fécondation d'un oeuf, dans laquelle le au moins un agent comprend un anticorps anti-p53.

11. Méthode de sélection d'un agent candidat pour l'aptitude à moduler la viabilité d'un embryon ou d'un précurseur d'embryon, dans laquelle le précurseur d'embryon est un oeuf fécondé, un zygote ou une entité pré-embryonnaire qui suit la fécondation d'un oeuf, la méthode comprenant les étapes qui consistent à mettre un premier embryon non humain ou un précurseur d'embryon non humain en contact avec l'agent candidat, à cultiver le premier embryon ou précurseur d'embryon *in vitro* dans le milieu de culture, à cultiver un second embryon non humain ou un précurseur d'embryon non humain qui n'a pas été exposé à l'agent candidat *in vitro* dans le milieu de culture, à mesurer le taux d'un marqueur qui est indicatif de la viabilité de l'embryon dans le milieu de culture du premier et du second embryon ou précurseur d'embryon, à comparer le taux mesuré du marqueur dans le milieu de culture du premier et du second embryon ou précurseur d'embryon et à déterminer ainsi si l'agent candidat module la viabilité de l'embryon, dans laquelle le marqueur est p53.

12. Méthode de sélection d'un agent candidat pour l'aptitude à moduler la viabilité d'un embryon ou d'un précurseur d'embryon, dans laquelle le précurseur d'embryon est un oeuf fécondé, un zygote ou une entité pré-embryonnaire qui suit la fécondation d'un oeuf, la méthode comprenant les étapes qui consistent à mettre un embryon non humain ou un précurseur d'embryon non humain en contact avec l'agent candidat, à cultiver l'embryon ou précurseur d'embryon *in vitro* dans le milieu de culture, à comparer le taux mesuré du marqueur dans le milieu de culture avec au moins un taux qui est indicatif de la viabilité de l'embryon et à déterminer ainsi si l'agent candidat module la viabilité de l'embryon, dans laquelle le marqueur est p53.

13. Méthode selon la revendication 11 ou 12, dans laquelle l'agent candidat est un milieu de culture.

14. Méthode selon la revendication 11 ou 12, dans laquelle la modulation de la viabilité de l'embryon est une augmentation de la viabilité dudit embryon.

15. Méthode selon l'une quelconque des revendications 1, 4, 11 ou 12, dans laquelle l'embryon ou le précurseur d'embryon est un embryon ou un précurseur d'embryon d'une espèce de primate, équine, bovine, ovine, caprine, léporine, aviaire, féline, canine ou murine non humaine.

16. Méthode selon la revendication 1 ou 4, dans laquelle l'embryon est un embryon humain.
